# FASCICULE DE BREVET EUROPEEN

(11) **EP 3 856 020 B1**
(45) Date de publication et mention de la délivrance du brevet: **09.11.2022**
(21) Numéro de dépôt: 19839338.1
(22) Date de dépôt: 26.09.2019
(51) Int. Cl.: A61B 5/022, A61B 5/021

(54) **SYSTEME DE CONNECTIQUE POUR SPHYGMOMANOMETRE**
VERBINDUNGSSYSTEM FÜR EIN SPHYGMOMANOMETER
CONNECTION SYSTEM FOR A SPHYGMOMANOMETER

(30) Priorité: 26.09.2018 FR 1858820
(43) Date de publication de la demande: 04.08.2021
(73) Titulaire: Spengler SAS, 13290 Aix-en-Provence (FR)
(72) Inventeur: BRULET, Nicolas, 13860 PEYROLLES EN PROVENCE (FR); JOUFFRET, Patrick, 83000 TOULON (FR)
(74) Mandataire: Roman, Alexis
(86) Numéro de dépôt international: PCT/FR2019/052290
(87) Numéro de publication internationale: WO 2020/065239

(56) Documents cités:
- EP-A1- 0 365 860
- EP-B1- 0 365 860
- FR-A1- 3 051 347
- US-A1- 2011 046 494
- US-A1- 2012 203 120

## Description

### Domaine technique de l'invention.

L'invention a pour objet un système de connectique pour sphygmomanomètre.

Elle concerne le domaine technique des dispositifs médicaux, et plus particulièrement celui des dispositifs permettant de mesurer la tension artérielle.

### État de la technique.

La mesure de la pression artérielle (ou tension artérielle) est une des mesures les plus courantes effectuées par les médecins. Une mesure rapide et précise est donc bénéfique que ce soit pour le médecin ou pour le patient. La méthode préférée pour effectuer une telle mesure reste l'utilisation d'un tensiomètre (ou sphygmomanomètre).

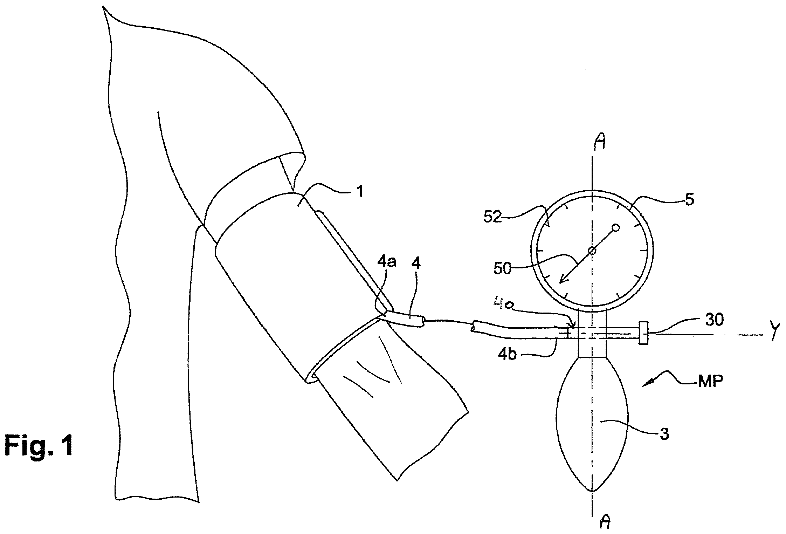

En se rapportant à la figure 1, cette mesure de la pression artérielle est généralement effectuée au moyen :
- d'un brassard gonflable 1 adapté pour être placé autour du membre (ici le bras) d'un patient à examiner et/ou à surveiller, lequel brassard comporte une poche gonflable,
- d'un système de mesure, comme par exemple un manomètre 2 de lecture de la pression artérielle qui est relié au brassard 1,
- d'une poire gonflable 3, elle-même reliée au brassard 1 par un cordon tubulaire souple 4, servant à augmenter la pression d'air dans ledit brassard. Cette poire 3 est généralement équipée d'une soupape dont le dispositif de commande 30 permet de contrôler la pression et de la faire diminuer progressivement de manière à effectuer la mesure.

Différents types de sphygmomanomètres ont été développés, dont le mano-poire MP illustré sur la figure 1. Le manomètre 5 et la poire de gonflage 3 sont réunis ensemble. Le cordon tubulaire souple 4 relie le mano-poire MP au brassard 1 pour assurer le gonflage de ce dernier.

Le brassard 1 doit être positionné autour du membre, de sorte que sa poche gonflable soit positionnée au niveau d'une artère. La poche est gonflée pour amener d'abord la pression du brassard 1 à une valeur supérieure à la pression systolique pour bloquer la circulation artérielle dans le membre. En réglant le dispositif de commande 30, la pression du brassard 1 est ensuite diminuée progressivement jusqu'à la valeur limite à partir de laquelle la pression artérielle est suffisante pour laisser de nouveau passer le sang dans l'artère. C'est la pression systolique. En poursuivant le dégonflage, on amène la pression du brassard 1 à une valeur à partir de laquelle il n'y a plus d'obstacle au flux artériel même lorsque le cœur est en diastole. C'est la pression diastolique.

Il existe de nombreuses causes pouvant amener à une mauvaise mesure de pression artérielle. Le choix du sphygmomanomètre utilisé est donc primordial pour que le diagnostic ne soit pas erroné.

L'ergonomie d'un mano-poire MP et/ou le système d'affichage du manomètre 5 (cadran à aiguille, affichage digital, ...) peuvent changer d'une marque à l'autre ou selon les modèles de mano-poires. Egalement, les dimensions du brassard 1 doivent être proportionnées à la morphologie du membre du patient, et notamment à sa circonférence. Un brassard trop étroit surestimera la pression artérielle et un brassard trop large la sous-estimera. Dans ces deux cas, la valeur de la pression artérielle obtenue n'est pas représentative et ne permet pas d'effectuer un diagnostic exact. Le choix de la taille du brassard utilisé est donc primordial pour que le diagnostic ne soit pas erroné. Il apparaît donc avantageux qu'un médecin puisse facilement changer de mano-poire et/ou de brassard selon ses besoins.

On connait par de document brevet FR3051347 (SPENGLER), un système de connectique pour sphygmomanomètre comprenant :
- un brassard gonflable,
- un cordon tubulaire souple comportant une première extrémité et une seconde extrémité, la première extrémité étant en communication fluidique avec la poche gonflable du brassard,
- un mano-poire,
- un organe de connexion monté dans le mano-poire pour connecter la seconde extrémité du cordon souple audit mano-poire,
- la seconde extrémité du cordon comporte un embout mâle et l'organe de connexion comporte un orifice femelle de connexion pour accueillir de manière démontable ledit embout mâle,
- l'embout mâle comporte plusieurs joints toriques espacés longitudinalement, lesquels joints coopèrent avec la paroi interne de l'orifice femelle de connexion pour former étanchéité à l'air.

Un tel système de connectique permet d'avoir un sphygmomanomètre à configuration interchangeable. Le médecin peut maintenant très facilement changer de mano-poire tout en utilisant le même brassard, ou inversement pour s'adapter à la morphologie du patient. Toutefois, dans la pratique, la connexion de l'embout mâle dans l'orifice femelle de connexion peut être relativement longue. De plus, une déconnexion du câble suite à un arrachement intempestif du cordon ne peut être exclue. Cette connexion peut en outre s'avérer malaisée du fait de la présence des joints toriques sur l'embout mâle et du frottement qu'ils génèrent. En outre, ces joints peuvent se dégrader lorsque l'embout mâle n'est pas connecté dans l'orifice femelle de connexion, et laissé libre dans l'environnement extérieur, ce qui peut, à terme, poser des problèmes d'étanchéité à l'air.

Un objectif de l'invention est de proposer un système de connectique pour sphygmomanomètre qui facilite l'assemblage entre l'organe de connexion du mano-poire et la seconde extrémité du cordon souple, de manière à changer encore plus rapidement de mano-poire et/ou de brassard.

Un autre objectif de l'invention est de proposer un système de connectique pour sphygmomanomètre dont la conception est particulièrement robuste.

Encore autre objectif de l'invention est de proposer un système de connectique pour sphygmomanomètre dont la conception est simple, peu onéreuse et qui soit simple à utiliser.

### Divulgation de l'invention.

La solution proposée par l'invention est un système de connectique pour sphygmomanomètre comprenant :
- un brassard gonflable adapté pour être placé sur le membre d'un patient à examiner et/ou à surveiller, lequel brassard comporte une poche gonflable,
- un cordon tubulaire souple comportant une première extrémité et une seconde extrémité, la première extrémité étant en communication fluidique avec la poche gonflable du brassard,
- un mano-poire formé par une poire de gonflage associée à un manomètre pour mesurer la pression artérielle,
- un organe de connexion monté dans le mano-poire pour connecter la seconde extrémité du cordon audit mano-poire, de sorte que ledit cordon soit adapté pour assurer une liaison directe entre ledit mano-poire et le brassard,
- la seconde extrémité du cordon comporte un embout mâle et l'organe de connexion comporte un orifice femelle de connexion pour accueillir de manière démontable ledit embout mâle.

Ce système est remarquable en ce que :
- l'organe de connexion comprend une portion qui coopère par aimantation avec une portion complémentaire aménagée sur l'embout mâle du cordon,
- au moins une des portions intègre un aimant permanent assurant l'aimantation.

La connexion aimantée entre le cordon et le mano-poire offre une solution simple et robuste qui permet de changer très simplement et très rapidement de mano-poire et/ou de brassard. En outre, la force d'alimentation permet de réduire les risques de déconnexion intempestive du câble.

D'autres caractéristiques avantageuses de l'invention sont listées ci-dessous. Chacune de ces caractéristiques peut être considérée seule ou en combinaison avec les caractéristiques remarquables définies ci-dessus, et faire l'objet, le cas échéant, d'une ou plusieurs demandes de brevet divisionnaires :
- Selon un mode de réalisation, un bloc de jonction est monté dans le mano-poire, lequel bloc de jonction est adapté pour assurer une communication fluidique entre la poire de gonflage, le manomètre, un dispositif de commande d'une soupape, et l'organe de connexion. L'organe de connexion est une pièce d'interface rapportée insérée dans une extrémité du bloc de jonction.
- Selon une variante de réalisation, un bloc de jonction est monté dans le mano-poire, lequel bloc de jonction est adapté pour assurer une communication fluidique entre la poire de gonflage, le manomètre, un dispositif de commande d'une soupape, et l'organe de connexion. L'organe de connexion fait partie intégrante du bloc de jonction et forme avec celui-ci une pièce monobloc.
- Avantageusement, la portion de l'organe de connexion se présente sous la forme d'une collerette.
- Avantageusement, la portion complémentaire aménagée sur l'embout mâle du cordon se présente sous la forme d'une collerette, laquelle collerette est disposée à l'extérieur dudit cordon et vient en appui contre la seconde extrémité dudit cordon.
- La collerette de l'embout mâle vient avantageusement en appui contre la collerette de l'organe de connexion, lorsque ledit embout mâle est connecté dans l'orifice femelle dudit organe de connexion.
- Selon un mode de réalisation, la portion de l'organe de connexion intègre un aimant permanent. Et la portion complémentaire aménagée sur l'embout mâle intègre un matériau ferromagnétique.
- Selon une variante de réalisation, la portion de l'organe de connexion intègre un matériau ferromagnétique. Et la portion complémentaire aménagée sur l'embout mâle intègre un aimant permanent.
- Selon une autre variante de réalisation, la portion de l'organe de connexion et la portion complémentaire aménagée sur l'embout mâle intègrent chacune un aimant permanent, lesdits aimants permanents étant agencés de sorte que leurs pôles de nature contraire soient en vis-à-vis et s'attirent.
- Avantageusement, la portion complémentaire aménagée sur l'embout mâle est une pièce rapportée et solidarisée audit embout mâle.
- Selon une variante de réalisation, la portion complémentaire aménagée sur l'embout mâle fait partie intégrante dudit embout mâle et forme avec celui-ci une pièce monobloc.
- Selon un mode de réalisation, un bloc de jonction est monté dans le mano-poire, lequel bloc de jonction est adapté pour assurer une communication fluidique entre la poire de gonflage, le manomètre, un dispositif de commande d'une soupape, et l'organe de connexion. Ce bloc de jonction est pourvu d'un joint d'étanchéité coopérant avec l'embout mâle du cordon pour assurer une étanchéité à l'air entre ledit élément mâle et ledit bloc de jonction.
- Selon une variante de réalisation, l'orifice femelle de connexion de l'organe de connexion est pourvu d'un joint d'étanchéité coopérant avec l'embout mâle du cordon pour assurer une étanchéité à l'air entre ledit élément mâle et ledit orifice femelle.
- Avantageusement, l'embout mâle du cordon présente une paroi extérieure dépourvue d'élément d'étanchéité à l'air.
- Avantageusement, un organe de connexion est monté dans le brassard pour connecter la première extrémité du cordon audit brassard. La première extrémité du cordon comporte un embout mâle et l'organe de connexion du brassard comporte un orifice femelle de connexion pour accueillir de manière démontable ledit embout mâle. L'organe de connexion du brassard comprend une portion qui coopère par aimantation avec une portion complémentaire aménagée sur l'embout mâle de la première extrémité du cordon. Au moins une desdites portions comporte un aimant permanent assurant l'aimantation.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 est une représentation schématique d'un système selon l'invention où le brassard est installé sur le bras d'un patient,
- la figure 2 est une vue en perspective illustrant la connexion de la seconde extrémité du cordon sur un mano-poire,
- la figure 3 est une vue schématique en coupe verticale d'un sphygmomanomètre selon l'invention,
- la figure 4 est une représentation en coupe d'un embout mâle du cordon et de l'organe de connexion monté dans le mano-poire,
- la figure 5 montre, de manière désassemblée, les éléments de connexion de la figure 4,
- la figure 6a est une représentation en coupe d'un embout mâle du cordon et de l'organe de connexion monté dans le mano-poire, dans une variante de réalisation,
- la figure 6b est une représentation en coupe d'un embout mâle du cordon et de l'organe de connexion monté dans le mano-poire, dans une autre variante de réalisation,
- la figure 7 est une représentation schématique des deux extrémités d'un cordon selon l'invention,
- la figure 8 est une représentation schématique d'une variante de réalisation d'un système selon l'invention où le brassard est installé sur le bras d'un patient,
- la figure 9 est une vue en perspective illustrant la connexion de la première extrémité du cordon sur un brassard.

### Modes préférés de réalisation de l'invention.

En se rapportant à la figure 1, le système objet de l'invention comporte les éléments suivants :
- un brassard gonflable 1 adapté pour être placé sur le bras d'un patient à examiner et/ou à surveiller, lequel brassard comporte une poche gonflable,
- un moyen de gonflage 3, tel qu'une poire de gonflage, associé à un cordon tubulaire souple 4 pour assurer le gonflage du brassard 1,
- un manomètre 5 pour mesurer la pression artérielle systolique et diastolique du patient, lequel manomètre est associé à la poire de gonflage 3 pour former un mano-poire MP.

Sur la figure 1, le brassard 1 est installé autour du bras d'un patient, au niveau de l'artère, de manière à effectuer les différentes mesures de tensions artérielles. Le cordon souple 4 permet d'amener l'air pour le gonflage du brassard 1. Lorsqu'il est gonflé, le brassard 1 écrase l'artère sur laquelle il est placé. En écoutant, à l'aide d'un stéthoscope, le bruit émis par le sang lors de son passage dans l'artère, deux valeurs de pression artérielle sont mesurées. Ainsi, lorsque le brassard 1 est suffisamment gonflé pour comprimer l'artère, le sang ne peut plus passer et le médecin ne perçoit pas de bruit. Le brassard 1 se dégonfle ensuite progressivement, permettant de percevoir le bruit des battements cardiaques définissant la pression artérielle systolique (Pression maximale). Ce bruit s'amplifie puis disparaît, la pression mesurée correspondant alors à la pression artérielle diastolique (Pression minimale).

Le brassard 1 peut être de différentes tailles de manière à pouvoir s'adapter à des patients aux morphologies et diamètre de bras différents. Il a par exemple une longueur comprise entre 40 cm et 100 cm, une largeur pouvant varier entre 5 cm et 30 cm. Le brassard 1 est fabriqué de manière conventionnelle et comporte une poche gonflable. Il peut être fabriqué en une seule pièce, avec une double paroi délimitant la poche gonflable, et être réalisé dans un matériau plastique souple imperméable tel qu'un polymère ou un élastomère, comme par exemple le polyéthylène ou encore le polyuréthane. De préférence, il est fabriqué dans un matériau thermoplastique. Le brassard 1 peut également être formé par deux parois en textile non imperméable, tel que le coton, lesquelles parois délimitent une poche dans laquelle est logée la poche gonflable, laquelle poche gonflable est réalisée dans un matériau plastique souple imperméable tel qu'un polymère ou un élastomère.

Un moyen de gonflage 3 permet d'injecter de l'air dans la poche gonflable du brassard 1 pour le gonfler. Sur la figure 1, ce moyen de gonflage 3 se présente préférentiellement sous la forme d'une poire de gonflage, laquelle poire est du type habituellement utilisé dans les systèmes de sphygmomanomètre connus de l'art antérieur. Il suffit au médecin d'exercer, à la main, des pressions successives sur la poire 3 pour injecter de l'air dans le cordon 4 et gonfler le brassard 1. La poire 3 est associée à un organe 30 de commande d'une valve ou d'une soupape de mise à l'air libre de la poche gonflable du brassard 1. L'actionnement de cet organe 30 permet notamment de réduire graduellement la pression du brassard 1 gonflé en vue de la détermination des pressions systoliques. Cet organe 30 peut se présenter sous la forme d'une molette ou d'un bouton poussoir.

Le manomètre 5 illustré sur la figure 1 présente une aiguille 50 coopérant avec une graduation 52 d'un cadran de lecture, pour indiquer la pression artérielle. Le manomètre 5 peut toutefois être pourvu d'un écran à affichage digital.

Le cordon tubulaire 4 est réalisé dans un matériau souple, comme par exemple un polyéthylène, un polystyrène, ou en tout autre matériau plastique convenant à l'homme du métier. Il a un diamètre pouvant varier de 0.2 cm à 2 cm, et une longueur comprise entre 20 cm et 2 m. Il comporte une première extrémité 4a et une seconde extrémité 4b. La première extrémité 4a est en communication fluidique avec la poche gonflable du brassard 1. Cette première extrémité 4a peut être directement soudée sur la poche gonflable du brassard 1, sans possibilité de déconnexion.

La figure 1 schématise une configuration où le manomètre 5 est associé à la poire de gonflage 3 pour former un mano-poire MP. Ce dispositif unique MP est connu de l'homme du métier. Le mano-poire MP est relié au brassard 1 au moyen du cordon 4 permettant à l'air de circuler depuis la poire 3 jusqu'à l'intérieur de la poche gonflable dudit brassard.

Le système comporte des moyens de connexion démontables constitués d'aménagements en forme d'embouts mâles et orifices femelles, du type assemblage rapide.

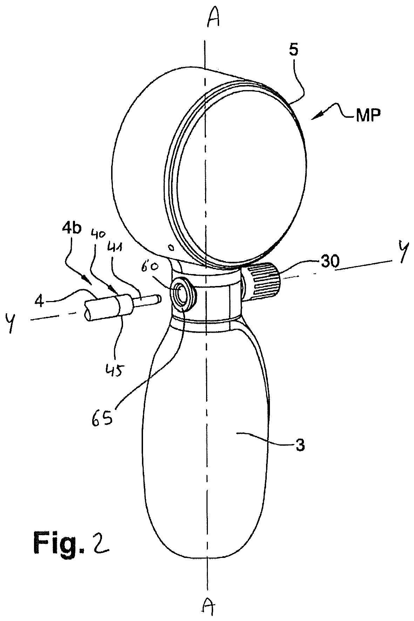

Sur les figures 2 à 7, le cordon 4 comporte des moyens de connexion 40 situés sur au moins la seconde extrémité 4b et préférentiellement sur chacune de ses extrémités 4a et 4b. Ces moyens de connexion 40 permettent notamment de raccorder le cordon 4 au mano-poire MP et éventuellement au brassard 1.

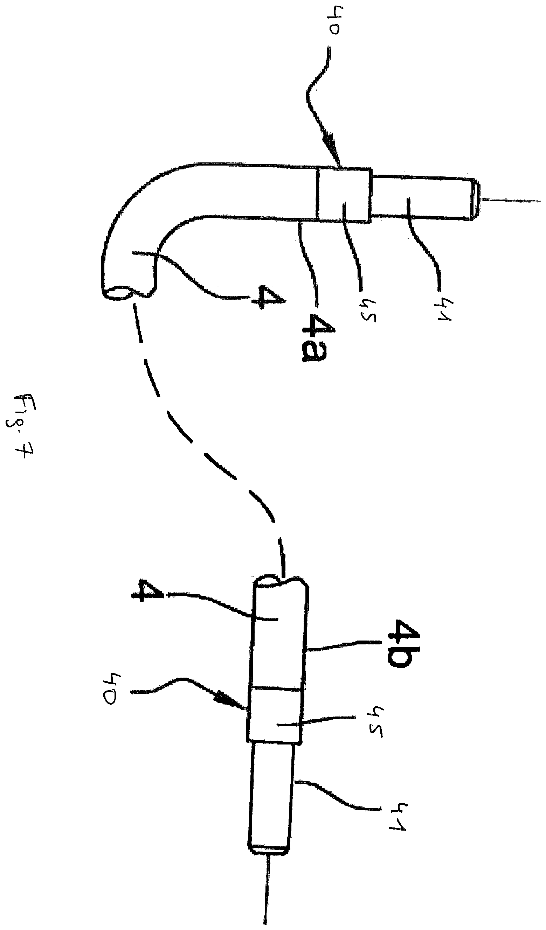

Sur la figure 7, le cordon 4 comporte un embout mâle 41 à chaque extrémité 4a, 4b. Ces embouts mâles 41 sont adaptés pour s'insérer dans des orifices femelles de connexion 60 complémentaires décrits plus avant dans la description et qui sont situés sur le mano-poire MP et éventuellement sur le brassard 1.

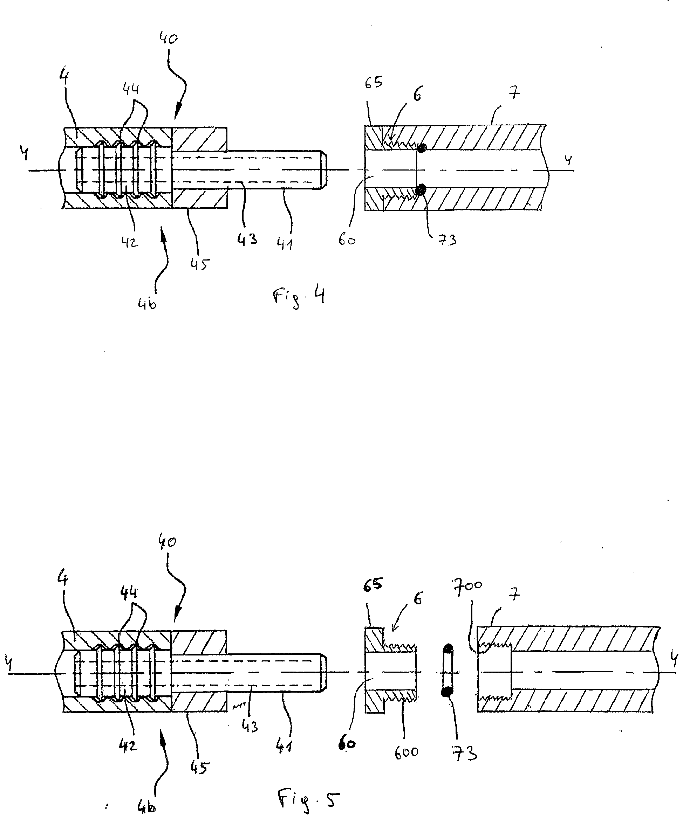

En se rapportant plus particulièrement à la figure 4, le moyen de connexion 40 est formé de l'embout mâle externe 41 et d'un embout mâle interne 42, ces deux embouts étant reliés par une portion 45, qui a préférentiellement la forme d'une collerette. Cette forme en collerette facilite notamment la manipulation du moyen de connexion 40 et simplifie sa conception. Dans la suite de la description, le terme « collerette » est employé comme synonyme de « portion ».

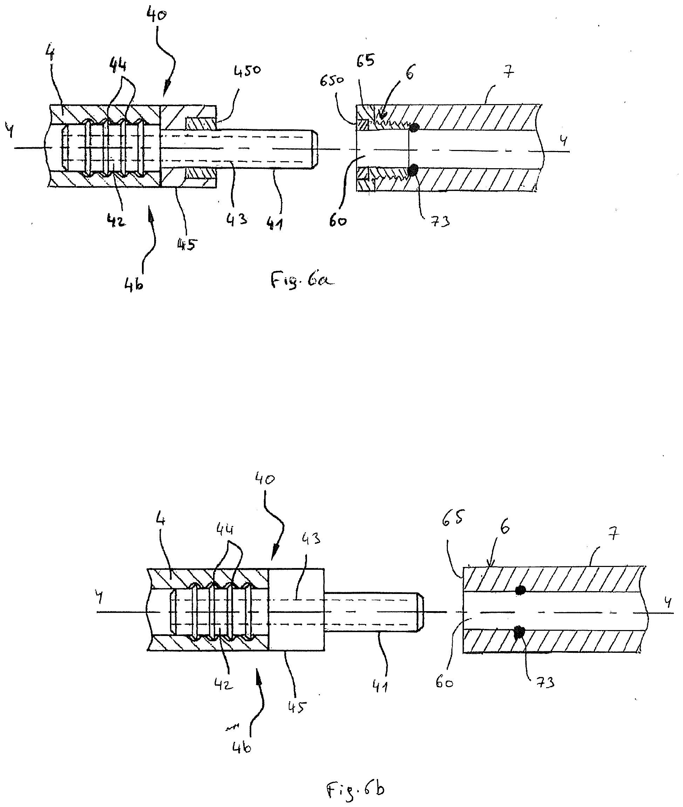

Pour simplifier davantage sa conception, ce moyen de connexion 40 peut être avantageusement fabriqué d'un seul tenant. En particulier, la collerette 45 fait partie intégrante de l'embout mâle externe 41 (et le cas échéant de l'embout mâle interne 42) et forme une pièce monobloc avec ledit embout mâle externe (figure 6b).

Les embouts 41, 42 et la collerette 45 peuvent être fabriqués dans un matériau ferromagnétique, comme par exemple un acier, ou tout autre matériau capable d'être attiré par un aimant. Dans une variante de réalisation, les embouts 41, 42 et la collerette 45 sont fabriqués dans un matériau magnétique pour former un aimant permanent. On utilise préférentiellement un matériau magnétique de la famille suivante : Néodyme-Fer-Bore, Samarium-Cobalt, alnicos, ferrite dur, alliage à base de Magnétite.

Sur les figures 4 et 5, le moyen de connexion 40 est fabriqué en deux parties, la collerette 45 étant une pièce rapportée et solidarisée à l'embout mâle externe 41. Cette solidarisation se fait par exemple par collage, vissage ou emmanchement forcé. Cette solution présente l'avantage de pouvoir utiliser deux matériaux distincts : par exemple un plastique tel que l'ABS pour les embouts 41, 42 et un matériau ferromagnétique ou un matériau magnétique pour la collerette 45. Lorsque la collerette 45 est réalisée dans un matériau magnétique, elle forme intégralement l'aimant permanent.

Sur la figure 6a, la collerette 45 comporte un aménagement dans lequel sont installés un ou plusieurs éléments ferromagnétiques, ou un ou plusieurs aimants permanents 450. La collerette 45 peut dans ce cas être réalisée en plastique, tel que l'ABS, par exemple.

Les embouts 41, 42 et la collerette 45 sont percés axialement de manière à former un conduit 43 autorisant la circulation d'air.

L'embout mâle externe 41 se présente, de préférence, sous la forme d'un cylindre. Ce cylindre a par exemple un diamètre externe compris entre 0,15 cm et 2 cm, et une longueur par exemple comprise entre 0,5 cm et 2 cm. La paroi extérieure de l'embout 41 est lisse, régulière, dépourvue d'élément d'étanchéité à l'air.

L'embout mâle interne 42 se présente, de préférence, sous la forme d'un cylindre similaire à celui formant l'élément mâle externe 41. Ce cylindre a par exemple un diamètre compris entre 0,15 cm et 2 cm, et une longueur pouvant par exemple varier de 0,5 cm à 2 cm.

L'embout mâle interne 42 est adapté pour s'insérer de manière étanche dans l'extrémité respective 4a, 4b du cordon tubulaire 4 de sorte que le moyen de connexion 40 soit solidarisé à ce dernier. Cet embout mâle interne 42 comporte au moins un collet d'accroche 44 coopérant avec la paroi interne du cordon 4. Ce collet d'accroche 44 permet de l'insérer en force dans le cordon tubulaire 4 et ainsi assurer l'étanchéité à l'air entre le moyen de connexion 40 et ledit cordon. Avantageusement, au moins deux collets 44, et préférentiellement au moins quatre collets d'accroché 44, sont disposés autour du cylindre de l'embout 42. En pratique, le diamètre externe des collets 44 est légèrement supérieur au diamètre interne du cordon 4 (par exemple de 1 mm) pour assurer l'insertion en force de l'embout mâle interne 42 et une déformation plastique de la paroi interne du cordon 4, laquelle déformation assure l'étanchéité à l'air.

La collerette 45 est disposée à l'extérieur du cordon 4 et vient en appui contre l'extrémité respective 4a, 4b de ce dernier. La collerette 45 se présente par exemple sous la forme d'un cylindre dont le diamètre externe correspond au diamètre externe du cordon 4, sa longueur étant par exemple comprise entre 0,5 cm et 1 cm.

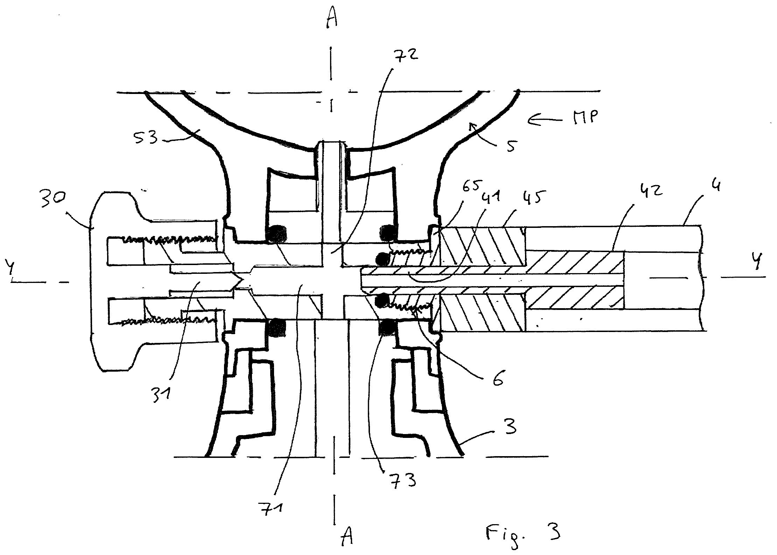

En se rapportant à la figure 3, un organe de connexion 6 est monté dans le mano-poire MP pour connecter la seconde extrémité 4b du cordon 4 audit mano-poire, de sorte que ledit cordon soit adapté pour assurer une liaison directe entre ledit mano-poire et le brassard 1. L'orifice femelle 60 est réalisé dans cet organe de connexion 6 et est adapté pour accueillir de manière démontable l'embout mâle 41. Comme cela apparaît sur la figure 3, l'entrée de l'orifice femelle 60 est préférentiellement aménagée dans la bordure latérale du mano-poire MP, de manière à ce que son axe Y-Y soit perpendiculaire à l'axe longitudinal A-A dudit mano-pore. La demanderesse a constaté que cette orientation permet un raccordement plus aisé du cordon 4.

Sur la figure 4, l'orifice femelle 60 présente une paroi cylindrique interne adaptée à la structure de l'élément mâle externe 41. Plus particulièrement, le cylindre de l'embout mâle externe 41 a un diamètre externe ajusté au diamètre interne de l'orifice femelle 60. Par « ajusté », on entend que le diamètre interne de l'orifice femelle 60 est sensiblement égal au diamètre externe du cylindre formant l'embout mâle externe 41, et préférentiellement légèrement supérieur (par exemple de 0,5 mm) pour éviter les frottements lors de l'insertion dudit embout 41. L'orifice femelle 60 a par exemple un diamètre interne compris entre 0,15 cm et 2 cm, et une longueur correspondant sensiblement à celle du cylindre formant l'embout mâle externe 41, ledit embout mâle étant toutefois légèrement plus long de sorte qu'il dépasse dudit orifice femelle 60 (figure 3).

L'organe de connexion 6 comprend une portion 65 qui coopère avec la portion complémentaire 45 aménagée sur l'embout mâle 71. La portion 65 se présente avantageusement sous la forme d'une collerette

Comme illustrée sur la figure 2, cette collerette 65 est située sur la paroi du mano-poire MP et donc parfaitement accessible. La collerette 65 se présente par exemple sous la forme d'un cylindre dont le diamètre externe correspond au diamètre externe de la collerette 45, sa longueur étant par exemple comprise entre 0,5 cm et 1 cm. Lorsque l'embout mâle 41 est connecté dans l'orifice femelle 60, les collerettes 45, 65 sont en appui l'une contre l'autre.

La collerette 65 peut être réalisée dans un matériau ferromagnétique ou un matériau magnétique. Lorsque la collerette 45 est réalisée dans un matériau magnétique, elle forme intégralement un aimant permanent.

Sur la figure 6a, la collerette 65 comporte un aménagement dans lequel sont installés un ou plusieurs éléments ferromagnétiques, ou un ou plusieurs aimants permanents 650. La collerette 65 peut dans ce cas être réalisée en plastique, tel que l'ABS, par exemple.

Pour assurer une connexion efficace entre le moyen de connexion 40 du cordon 4 et l'organe de connexion 6, les portions 45 et 65 coopèrent par aimantation. Si la collerette 45 intègre un matériau ferromagnétique, la collerette 65 intègre un aimant permanent. Si la collerette 45 intègre un aimant permanent, la collerette 65 intègre un matériau ferromagnétique. La collerette 45 et la collerette 65 peuvent également toutes deux intégrer un aimant permanent. Dans ce dernier cas, les aimants permanents sont agencés de sorte que leurs pôles de nature contraires soient en vis-à-vis et s'attirent.

La collerette 45 exerce une force d'attraction sur la collerette 65 (ou inversement) qui permet d'insérer automatiquement l'élément mâle 41 dans l'organe femelle 60, sans force ou intervention supplémentaire de l'utilisateur. Dès que l'élément mâle 41 est engagé dans l'organe femelle 60, la connexion se fait toute seule, jusqu'à ce que la collerette 45 vienne en butée contre la collerette 65. Lorsque les collerettes 45, 65 sont en appui l'une contre l'autre, la force d'aimantation assure le maintien en position de l'élément mâle 41 dans l'organe femelle 60, permettant ainsi de réduire les risques de déconnexion du câble 4 lors d'un arrachement intempestif. Avec une force d'adhérence comprise entre 1 kg et 3 kg (soit environ 10-30 newtons) entre les collerettes 45 et 65, on obtient un bon compromis entre : réduction des risques de déconnexion intempestive du câble 4 et déconnexion volontaire aisée dudit câble.

Sur la figure 3, un bloc de jonction 7 est installé à l'intérieur de la coque 53 du mano-poire MP, dans un alésage axial. Ce bloc de jonction 7 assure une communication fluidique entre la poire 3, le manomètre 5, une soupape 31 du dispositif de commande 30, et l'organe de connexion 6 du cordon 4. Ce bloc de jonction 7 est similaire à celui décrit dans le document brevet FR3051344 (SPENGLER) auquel l'homme du métier pourra se référer en cas de besoin.

Ce bloc 7 a une forme extérieure générale cylindrique. Il est avantageusement réalisé dans un matériau rigide, préférentiellement un plastique tel que l'ABS, ou un métal, tel que l'inox. Il est percé axialement de manière à former un canal 71 autorisant la circulation d'air. Le bloc 7 a par exemple un diamètre interne compris entre 0,2 cm et 1 cm et un diamètre externe compris entre 0,4 cm et 1,5 cm. Sa longueur est par exemple comprise entre 1 cm et 3 cm.

Le canal 71 présente une première extrémité au niveau de laquelle est agencé l'organe de connexion 6, et une seconde extrémité dans laquelle est engagée la soupape 31 de dégonflage du brassard 1.

Le bloc 7 comporte également un alésage 72 qui s'étend selon l'axe vertical A-A du mano-poire MP et qui est en communication fluidique avec le canal 71. Cet alésage 72 présente une extrémité inférieure en communication fluidique avec la poire 3 et une extrémité supérieure en communication fluidique avec la chambre de mesure du manomètre 5.

Sur les figures 3, 4, 5 et 6a, l'organe de connexion 6 est une pièce d'interface rapportée, qui est insérée dans la première extrémité du bloc 7. Pour simplifier son assemblage, cette pièce d'interface 6 est préférentiellement vissée dans la première extrémité du bloc 7. Des solutions par soudage ou emmanchement forcé sont toutefois envisageables, bien que ces solutions présentent le désavantage de nécessiter des outillages et/ou étapes supplémentaires.

Dans une solution par vissage, la pièce d'interface 6 présente un corps cylindrique percé axialement et présentant un filetage extérieur 600 qui s'engage dans un filetage complémentaire 700 réalisé sur la paroi interne du bloc 7, au niveau de sa première extrémité. Ce corps cylindrique fileté porte, au niveau d'une de ses extrémités, la collerette 65. La pièce d'interface 6 est insérée dans le bloc 7 jusqu'à ce que la face arrière de la collerette 65 vienne en butée contre la première extrémité du bloc 7.

La figure 6b illustre une variante de réalisation où l'organe de connexion 6 fait partie intégrante du bloc 7 et forme avec celui-ci une pièce monobloc. La collerette 65 est dans ce cas une partie intégrante du bloc 7 située au niveau de la première extrémité de celui-ci.

Sur les figures 3 à 5 et 6b, le bloc 7 est pourvu d'au moins un joint torique 73 coopérant avec l'embout mâle 41 pour assurer une étanchéité à l'air entre ledit élément mâle et ledit bloc de jonction. Ce joint d'étanchéité 73 est disposé dans l'alésage axial du bloc 7. Il peut être logé dans une gorge dédiée prévue à cet effet dans la paroi interne dudit alésage, ou être maintenu en position par la pièce d'interface formant l'organe de connexion 6. Le joint 73 est préférentiellement réalisé en caoutchouc mais il peut également être réalisé dans un autre matériau, comme par exemple le silicone. Dans une variante de réalisation illustrée sur la figure 6a, le joint 73 est directement intégré dans l'orifice femelle de connexion 60 pour assurer une étanchéité à l'air entre l'élément mâle 41 et ledit orifice femelle.

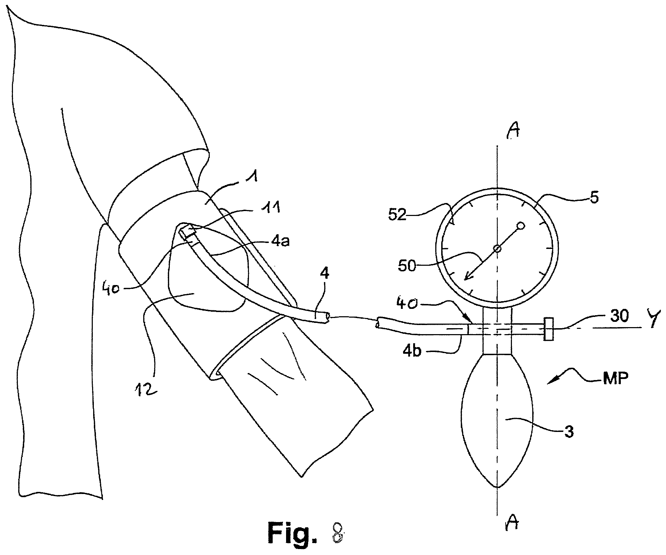

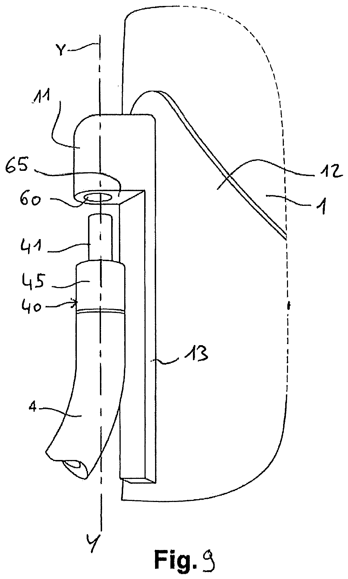

Les figures 8 et 9 illustrent le cas où la première extrémité 4a du cordon 4 est connectée de manière démontable au brassard 1, ce cordon assurant une liaison directe entre le mano-poire MP et ledit brassard. Dans ce cas, l'organe de connexion 6 est monté dans le brassard 1, et plus particulièrement aménagé dans une protubérance 11. Son orifice femelle 60, en communication fluidique avec la poche gonflable du brassard 1, est adapté pour accueillir de manière démontable l'embout mâle 41 de la première extrémité 4a.

La protubérance 11 est façonnée en saillie sur une pièce d'interface 12, laquelle pièce d'interface fait office d'interface entre la poche gonflable et l'orifice femelle 60 du brassard 1. La protubérance 11 est avantageusement réalisée dans un matériau rigide, en plastique ou en métal. Elle pourrait toutefois être réalisée dans un matériau plastique semi-rigide ou souple.

La face de la protubérance 11 sur laquelle est aménagée l'entrée de l'organe femelle 60, correspond à la portion 65 de l'organe de connexion 6 qui coopère par aimantation avec la collerette 45 de l'embout mâle 41. Aussi, comme décrit précédemment, la portion 65 peut intégrer un aimant permanent dans le cas où la collerette 45 est réalisée dans un matériau ferromagnétique ou dans le cas où ladite collerette 45 intègre également un aimant permanent. La portion 65 peut encore être réalisée dans un matériau ferromagnétique dans le cas la collerette 45 intègre un aimant permanent.

Un aménagement en relief 13, en forme de semelle, est installé devant l'orifice femelle 60 et dans l'axe Y-Y de ce dernier. Cette semelle 13 permet de guider en position l'embout mâle 41 du cordon 4. Cette semelle 13 permet également de rigidifier et de renforcer la pièce d'interface 12. Sa longueur est par exemple comprise entre 1 cm et 5 cm, sa largeur correspond préférentiellement à celle de la protubérance 11, et son épaisseur est par exemple comprise entre 2 mm et 1 cm. Cette semelle 13 peut présenter une concavité dans l'axe Y de manière à faciliter le guidage de l'embout 41.

L'utilisation du système objet de l'invention va maintenant être décrite en détail. Le médecin a à sa disposition : le brassard 1, un cordon 4 et le mano-poire MP.

Lorsque le médecin a besoin d'utiliser un mano-poire, il lui suffit de placer un brassard 1 de taille adaptée autour du bras du patient. Il connecte ensuite un des embouts mâles 41 du cordon 4 dans l'orifice femelle de connexion 60 du brassard 1 (figure 9) si la première extrémité 4a de ce dernier n'est pas déjà solidarisée audit brassard (figure 1). L'embout mâle 41 de la seconde extrémité 4b du cordon 4 est connecté dans l'orifice femelle de connexion 60 du mano-poire MP de son choix, par exemple avec un manomètre 5 à aiguille et/ou d'un certain modèle ou marque.

Lorsque le médecin a besoin d'utiliser un autre mano-poire (par exemple avec un manomètre 5 à affichage digital et/ou d'un autre modèle ou marque) il lui suffit de déconnecter l'embout mâle 41 de la seconde extrémité 4b hors de l'orifice femelle de connexion 60 du mano-poire MP. Il reconnecte ensuite cet embout mâle 41 dans l'orifice femelle de connexion 60 d'un autre mano-poire MP.

Le médecin peut également facilement utiliser un autre brassard 1, tout en conservant son mano-poire MP.

Si la première extrémité 4a du cordon 4 est solidarisée au brassard 1 à changer (configuration de la figure 1), il lui suffit de déconnecter l'embout mâle 41 de la seconde extrémité 4b hors de l'orifice femelle de connexion 60 du mano-poire MP. Il reconnecte ensuite l'embout mâle 41 de la seconde extrémité 4b du cordon 4 rattaché à un autre brassard 1, de taille plus adaptée, dans l'orifice femelle de connexion 60 du mano-poire MP.

Si la première extrémité 4a du cordon 4 est connectée de manière amovible au brassard 1 à changer (configuration des figures 8 et 9), il lui suffit de déconnecter l'embout mâle 41 de la première extrémité 4a hors de l'orifice femelle de connexion 60 dudit brassard. Il reconnecte ensuite cet embout mâle dans l'orifice femelle de connexion 60 d'un autre brassard 1 de taille plus adaptée. L'embout mâle 41 de la seconde extrémité 4b du cordon 4 reste connecté dans l'orifice femelle de connexion 60 du mano-poire MP.

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes. En particulier :
- Le brassard 1 peut être installé autour d'un membre approprié du patient tel que : bras, poignet, jambe, etc.
- Des moyens de gonflage 3 plus complexes qu'une poire peuvent être utilisés, comme par exemple une pompe à piston ou encore une pompe à engrenage.
- L'embout interne 42 et l'embout externe 41 peuvent être fabriqués séparément et assemblés entre eux ultérieurement par vissage ou grâce à un procédé de soudure ou de collage par exemple.
- La section du cylindre composant l'embout mâle externe 41 n'est pas forcément circulaire, elle peut par exemple être carrée, rectangulaire ou encore elliptique.
- Le brassard 1 ne comporte pas forcément de pièce d'interface 12. L'orifice femelle 60 peut, par exemple, être agencé directement dans la poche gonflable. La protubérance 11 peut également être rattachée directement à la poche gonflable.

## Revendications

1. Système de connectique pour sphygmomanomètre comprenant :
- un brassard gonflable (1) adapté pour être placé sur le membre d'un patient à examiner et/ou à surveiller, lequel brassard comporte une poche gonflable,
- un cordon tubulaire souple (4) comportant une première extrémité (4a) et une seconde extrémité (4b), la première extrémité (4a) étant en communication fluidique avec la poche gonflable du brassard (1),
- un mano-poire (MP) formé par une poire de gonflage (3) associée à un manomètre (5) pour mesurer la pression artérielle,
- un organe de connexion (6) monté dans le mano-poire (MP) pour connecter la seconde extrémité (4b) du cordon (4) audit mano-poire (MP), de sorte que ledit cordon soit adapté pour assurer une liaison directe entre ledit mano-poire (MP) et le brassard (1),
- la seconde extrémité (4b) du cordon (4) comporte un embout mâle (41) et l'organe de connexion (6) comporte un orifice femelle de connexion (6) pour accueillir de manière démontable ledit embout mâle,
**se caractérisant par le fait que** :
- l'organe de connexion (6) comprend une portion (65) qui coopère par aimantation avec une portion complémentaire (45) aménagée sur l'embout mâle (71) du cordon (4),
- au moins une des portions (45, 65) intègre un aimant permanent assurant l'aimantation.

2. Système selon la revendication 1, **dans lequel** :
- un bloc de jonction (7) est monté dans le mano-poire (MP), lequel bloc de jonction est adapté pour assurer une communication fluidique entre la poire de gonflage (3), le manomètre (5), un dispositif de commande (30) d'une soupape (31), et l'organe de connexion (6),
- l'organe de connexion (6) est une pièce d'interface rapportée insérée dans une extrémité du bloc de jonction (7).

3. Système selon la revendication 1, **dans lequel** :
- un bloc de jonction (7) est monté dans le mano-poire (MP), lequel bloc de jonction est adapté pour assurer une communication fluidique entre la poire de gonflage (3), le manomètre (5), un dispositif de commande (30) d'une soupape (31), et l'organe de connexion (6),
- l'organe de connexion (6) fait partie intégrante du bloc de jonction (7) et forme avec celui-ci une pièce monobloc.

4. Système selon l'une des revendications précédentes, **dans lequel** la portion (65) de l'organe de connexion (6) se présente sous la forme d'une collerette.

5. Système selon l'une des revendications précédentes, **dans lequel** la portion complémentaire (45) aménagée sur l'embout mâle (41) du cordon (4) se présente sous la forme d'une collerette, laquelle collerette est disposée à l'extérieur dudit cordon et vient en appui contre la seconde extrémité (4b) dudit cordon.

6. Système selon les revendications 4 et 5 prises en combinaison, **dans lequel** la collerette (45) de l'embout mâle (41) vient en appui contre la collerette (65) de l'organe de connexion (6), lorsque ledit embout mâle est connecté dans l'orifice femelle (60) dudit organe de connexion.

7. Système selon l'une des revendications précédentes, **dans lequel** :
- la portion (65) de l'organe de connexion (6) intègre un aimant permanent,
- la portion complémentaire (45) aménagée sur l'embout mâle (41) intègre un matériau ferromagnétique.

8. Système selon l'une des revendications 1 à 6, **dans lequel** :
- la portion (65) de l'organe de connexion (6) intègre un matériau ferromagnétique,
- la portion complémentaire (45) aménagée sur l'embout mâle (41) intègre un aimant permanent.

9. Système selon l'une des revendications 1 à 6, **dans lequel** la portion (65) de l'organe de connexion (6) et la portion complémentaire (45) aménagée sur l'embout mâle (41) intègrent chacune un aimant permanent, lesdits aimants permanents étant agencés de sorte que leurs pôles de nature contraire soient en vis-à-vis et s'attirent.

10. Système selon l'une des revendications précédentes, **dans lequel** la portion complémentaire (45) aménagée sur l'embout mâle (41) est une pièce rapportée et solidarisée audit embout mâle.

11. Système selon l'une des revendications 1 à 9, **dans lequel** la portion complémentaire (45) aménagée sur l'embout mâle (41) fait partie intégrante dudit embout mâle et forme avec celui-ci une pièce monobloc.

12. Système selon l'une des revendications précédentes, **dans lequel** :
- un bloc de jonction (7) est monté dans le mano-poire (MP), lequel bloc de jonction est adapté pour assurer une communication fluidique entre la poire de gonflage (3), le manomètre (5), un dispositif de commande (30) d'une soupape (31), et l'organe de connexion (6),
- le bloc de jonction (7) est pourvu d'un joint d'étanchéité (73) coopérant avec l'embout mâle (41) du cordon (4) pour assurer une étanchéité à l'air entre ledit élément mâle et ledit bloc de jonction.

13. Système selon l'une des revendications 1 à 11, **dans lequel** l'orifice femelle de connexion (60) de l'organe de connexion (6) est pourvu d'un joint d'étanchéité (73) coopérant avec l'embout mâle (41) du cordon (4) pour assurer une étanchéité à l'air entre ledit élément mâle et ledit orifice femelle.

14. Système selon l'une des revendications précédentes, **dans lequel** l'embout mâle (71) du cordon (4) présente une paroi extérieure dépourvue d'élément d'étanchéité à l'air.

15. Système selon l'une des revendications précédentes, **dans lequel** :
- un organe de connexion (6) est monté dans le brassard (1) pour connecter la première extrémité (4a) du cordon (4) audit brassard,
- la première extrémité (4a) du cordon (4) comporte un embout mâle (41) et l'organe de connexion (6) du brassard (1) comporte un orifice femelle de connexion (60) pour accueillir de manière démontable ledit embout mâle,
- l'organe de connexion (6) du brassard (1) comprend une portion (65) qui coopère par aimantation avec une portion complémentaire (45) aménagée sur l'embout mâle (41) de la première extrémité (4a) du cordon (4),
- au moins une desdites portions (45, 65) comporte un aimant permanent assurant l'aimantation.

## Patentansprüche

1. Verbindungssystem für Blutdruckmessgerät, umfassend:
- eine aufblasbare Manschette (1), die ausgelegt ist, an die Extremität eines zu untersuchenden und/oder zu überwachenden Patienten angebracht zu werden, wobei die Manschette eine aufblasbare Tasche aufweist,
- ein flexibles Schlauchkabel (4), das ein erstes Ende (4a) und ein zweites Ende (4b) aufweist, wobei das erste Ende (4a) in fluidischer Kommunikation mit der aufblasbaren Tasche der Manschette (1) steht,
- eine Mano-Birne (MP), die von einer Inflationsbirne (3) gebildet wird, die mit einem Druckmesser (5) verbunden ist, um den Blutdruck zu messen,
- eine in der Mano-Birne (MP) montierte Verbindungsvorrichtung (6), um das zweite Ende (4b) des Kabels (4) mit der Mano-Birne (MP) zu verbinden, so dass das Kabel ausgelegt ist, um eine direkte Verbindung zwischen der Mano-Birne (MP) und der Manschette (1) zu gewährleisten,
- wobei das zweite Ende (4b) des Kabels (4) eine männliche Spitze (41) aufweist und die Verbindungsvorrichtung (6) eine weibliche Verbindungsöffnung (6) aufweist, um die männliche Spitze entfernbar aufzunehmen, **gekennzeichnet durch die Tatsache, dass:**
- die Verbindungsvorrichtung (6) einen Teil (65) umfasst, der durch Magnetisierung mit einem zusätzlichen Teil (45) zusammenarbeitet, der an der männlichen Spitze (71) des Kabels (4) angeordnet ist,
- mindestens einer der Teile (45, 65) einen Permanentmagneten enthält, der die Magnetisierung gewährleistet.

2. System nach Anspruch 1, **wobei:**
- ein Anschlussblock (7) in der Mano-Birne (MP) montiert ist, wobei der Anschlussblock ausgelegt ist, um eine fluidische Kommunikation zwischen der Aufblasbirne (3), dem Druckmesser (5), einer Regeleinrichtung (30) eines Ventils (31) und der Verbindungsvorrichtung (6) zu gewährleisten,
- die Verbindungsvorrichtung (6) ein angebrachter Schnittstellenteil ist, der in ein Ende des Anschlussblocks (7) eingeführt ist.

3. System nach Anspruch 1, **wobei:**
- ein Anschlussblock (7) in der Mano-Birne (MP) montiert ist, wobei der Anschlussblock ausgelegt ist, um eine fluidische Kommunikation zwischen der Aufblasbirne (3), dem Druckmesser (5), einer Regeleinrichtung (30) eines Ventils (31) und der Verbindungsvorrichtung (6) zu gewährleisten,
- die Verbindungsvorrichtung (6) ein integraler Bestandteil des Anschlussblocks (7) ist und mit ihm ein einteiliges Stück ausbildet.

4. System nach einem der vorhergehenden Ansprüche, **wobei** der Teil (65) der Verbindungsvorrichtung (6) in Form eines Kragens vorliegt.

5. System nach einem der vorhergehenden Ansprüche, **wobei** der zusätzliche Teil (45), der an der männlichen Spitze (41) des Kabels (4) angeordnet ist, in Form eines Kragens vorliegt, wobei der Kragen an der Außenseite des Kabels angeordnet ist und gegen das zweite Ende (4b) des Kabels in Anlage gelangt.

6. System nach den Ansprüchen 4 und 5 in Kombination genommen, wobei der Kragen (45) der männlichen Spitze (41) gegen den Kragen (65) der Verbindungsvorrichtung (6) in Anlage gelangt, wenn die männliche Spitze in der weiblichen Öffnung (60) der Verbindungsvorrichtung verbunden ist.

7. System nach einem der vorhergehenden Ansprüche, **wobei:**
- der Teil (65) der Verbindungsvorrichtung (6) einen Permanentmagneten enthält,
- der zusätzliche Teil (45), der an der männlichen Spitze (41) angeordnet ist, ein ferromagnetisches Material enthält.

8. System nach einem der Ansprüche 1 bis 6, **wobei:**
- der Teil (65) der Verbindungseinrichtung (6) ein ferromagnetisches Material enthält,
- der zusätzliche Teil (45), der an der männlichen Spitze (41) angeordnet ist, einen Permanentmagneten enthält.

9. System nach einem der Ansprüche 1 bis 6, **wobei** der Teil (65) der Verbindungsvorrichtung (6) und der zusätzliche Teil (45), der auf der männlichen Spitze (41) angeordnet ist, jeweils einen Permanentmagneten enthalten, wobei die Permanentmagnete so angeordnet sind, dass ihre Pole entgegengesetzter Natur entgegengesetzt sind und sich anziehen.

10. System nach einem der vorhergehenden Ansprüche, wobei der zusätzliche Teil (45), der auf der männlichen Spitze (41) angeordnet ist, ein Stück ist, das an der männlichen Spitze angebracht und befestigt ist.

11. System nach einem der Ansprüche 1 bis 9, **wobei** der zusätzliche Teil (45), der auf der männlichen Spitze (41) angeordnet ist, ein integraler Bestandteil der männlichen Spitze ist und mit ihr ein einteiliges Stück ausbildet.

12. System nach einem der vorhergehenden Ansprüche, **wobei:**
- ein Anschlussblock (7) in der Mano-Birne (MP) montiert ist, wobei der Anschlussblock ausgelegt ist, um eine fluidische Kommunikation zwischen der Aufblasbirne (3), dem Druckmesser (5), einer Regeleinrichtung (30) eines Ventils (31) und der Verbindungsvorrichtung (6) zu gewährleisten,
- der Anschlussblock (7) mit einer Dichtung (73) versehen ist, die mit der männlichen Spitze (41) des Kabels (4) zusammenarbeitet, um die Luftdichtheit zwischen dem männlichen Element und dem Anschlussblock zu gewährleisten.

13. System nach einem der Ansprüche 1 bis 11, **wobei** die weibliche Verbindungsöffnung (60) der Verbindungsvorrichtung (6) mit einer Dichtung (73) versehen ist, die mit der männlichen Spitze (41) des Kabels (4) zusammenarbeitet, um die Luftdichtheit zwischen dem männlichen Element und der weiblichen Öffnung zu gewährleisten.

14. System nach einem der vorhergehenden Ansprüche, **wobei** die männliche Spitze (71) des Kabels (4) eine Außenwand ohne Luftdichtheitselement aufweist.

15. System nach einem der vorhergehenden Ansprüche, **wobei:**
- eine Verbindungsvorrichtung (6) in der Manschette (1) montiert ist, um das erste Ende (4a) des Kabels (4) mit der Manschette zu verbinden,
- das erste Ende (4a) des Kabels (4) eine männliche Spitze (41) aufweist und die Verbindungsvorrichtung (6) der Manschette (1) eine weibliche Verbindungsöffnung (60) aufweist, um die männliche Spitze entfernbar aufzunehmen,
- die Verbindungsvorrichtung (6) der Manschette (1) einen Teil (65) umfasst, der durch Magnetisierung mit einem zusätzlichen Teil (45) zusammenarbeitet, der an der männlichen Spitze (41) des ersten Endes (4a) des Kabels (4) angeordnet ist,
- mindestens einer der Teile (45, 65) einen Permanentmagneten aufweist, der die Magnetisierung gewährleistet.

## Claims

1. A connection device for blood pressure monitor comprising:
- an adapted inflatable cuff (1) to be placed on the limb of a patient to be examined and/or monitored, said cuff comprising an inflatable pouch,
- a flexible tubular cord (4) comprising a first end (4a) and a second end (4b), the first end (4a) being in fluid communication with the inflatable pouch of the cuff (1),
- a mano-bulb (MB) formed by an inflating bulb (3) connected to a pressure gauge (5) for measuring blood pressure,
- a connecting element (6) mounted in the mano-bulb (MB) for connecting the second end (4b) of the cord (4) to said mano-bulb (MB), so that said cord is adapted to provide a direct connection between said bulb (MP) and the cuff (1),
- the second end (4b) of the cord (4) comprises a male end (41) and the connecting element (6) comprises a female connecting orifice (6) for removably receiving said male end, **characterised in that:**
- the connecting element (6) comprises a part (65) which cooperates by magnetisation with a complementary part (45) arranged on the male end (71) of the cord (4),
- at least one of the parts (45, 65) incorporates a fixed magnet ensuring magnetisation.

2. A device according to claim 1, **wherein:**
- a junction block (7) is mounted in the mano-bulb (MB), said junction block being adapted to provide fluid communication between the inflating bulb (3), the pressure gauge (5), a control device (30) of a valve (31), and the connecting element (6),
- the connecting element (6) is an interface insert inserted into one end of the junction block (7).

3. A device according to claim 1, **wherein:**
- a junction block (7) is mounted in the mano-bulb (MB), said junction block being adapted to provide fluid communication between the inflating bulb (3), the pressure gauge (5), a control device (30) of a valve (31), and the connecting element (6),
- the connecting element (6) is an integral part of the junction block (7) and forms an integrated part therewith.

4. A device according to one of the preceding claims, **wherein** the part (65) of the connecting element (6) takes the form of a flange.

5. A device according to one of the preceding claims, **wherein** the complementary part (45) arranged on the male end (41) of the cord (4) takes the form of a flange, which flange is arranged outside said cord and bears against the second end (4b) of said cord.

6. A device according to claims 4 and 5 taken in combination, **wherein** the flange (45) of the male end (41) bears against the flange (65) of the connecting element (6), when said male end is connected in the female orifice (60) of said connecting element.

7. A device according to one of the preceding claims, **wherein:**
- the part (65) of the connecting element (6) incorporates a fixed magnet,
- the complementary part (45) arranged on the male end (41) incorporates a ferromagnetic material.

8. A device according to one of claims 1 to 6, **wherein:**
- the part (65) of the connecting element (6) incorporates a ferromagnetic material,
- the complementary part (45) arranged on the male end (41) incorporates a fixed magnet.

9. A device according to one of claims 1 to 6, **wherein** the part (65) of the connecting element (6) and the complementary part (45) arranged on the male end (41) each incorporate a fixed magnet, said fixed magnets being arranged so that their opposite poles face each other and attract.

10. A device according to one of the preceding claims, **wherein** the complementary part (45) arranged on the male end (41) is an insert and integral with said male end.

11. A device according to one of claims 1 to 9, **wherein** the complementary part (45) arranged on the male end (41) is an integral part of said male end and forms an integrated part therewith.

12. A device according to one of the preceding claims, **wherein:**
- a junction block (7) is mounted in the mano-bulb (MB), said junction block being adapted to provide fluid communication between the inflating bulb (3), the pressure gauge (5), a control device (30) of a valve (31), and the connecting element (6),
- the junction block (7) is provided with a seal (73) cooperating with the male end (41) of the cord (4) to provide an airtight seal between said male element and said junction block.

13. A device according to one of claims 1 to 11, **wherein** the female connection orifice (60) of the connecting element (6) is provided with a seal (73) cooperating with the male end (41) of the cord (4) to provide an airtight seal between said male element and said female orifice.

14. A device according to one of the preceding claims, **wherein** the male end (71) of the cord (4) has an outer wall without an air-sealing element.

15. A device according to one of the preceding claims, **wherein:**
- a connecting element (6) is mounted in the cuff (1) to connect the first end (4a) of the cord (4) to said cuff,
- the first end (4a) of the cord (4) has a male end (41) and the connecting element (6) of the cuff (1) has a female connection orifice (60) for removably receiving said male end,
- the connecting element (6) of the cuff (1) comprises a part (65) which cooperates by magnetisation with a complementary part (45) arranged on the male end (41) of the first end (4a) of the cord (4),
- at least one of said parts (45, 65) comprises a fixed magnet ensuring the magnetisation.
